# EUROPÄISCHE PATENTSCHRIFT

(11) **EP 1 425 096 B1**
(45) Veröffentlichungstag und Bekanntmachung des Hinweises auf die Patenterteilung: **14.10.2009**
(21) Anmeldenummer: 02797635.6
(22) Anmeldetag: 28.08.2002
(51) Int. Cl.: B01J 23/889, B01J 37/03, C07C 209/26, C07C 209/48, C07C 253/30

(54) **ISOPHORONDIAMIN (IPDA, 3-AMINOMETHYL-3,5,5-TRIMETHYLCYCLOHEXYLAMIN)**
METHOD FOR THE PRODUCTION OF ISOPHORONDIAMINE (IPDA, 3-AMINOMETHYL-3,5,5-TRIMETHYLCYCLOHEXYLAMINE)
PROCEDE POUR PRODUIRE DE LA ISOPHORONDIAMINE (IPDA, 3-AMINOMETHYL-3,5,5-TRIMETHYLCYCLOHEXYLAMINE)

(30) Priorität: 31.08.2001 DE 10142635
(43) Veröffentlichungstag der Anmeldung: 09.06.2004
(73) Patentinhaber: BASF SE, 67056 Ludwigshafen (DE)
(72) Erfinder: FUNKE, Frank, 67067 Ludwigshafen 96 (DE); HILL, Thomas, 67051 Ludwigshafen (DE); MELDER, Johann-Peter, 67459 Böhl-Iggelheim (DE); SCHWAB. Ekkehard, 67343 Neustadt (DE); HIMMEL, Walter, 67269 Grünstadt (DE); HENKES, Erhard, 64683 Einhausen (DE); PETERSEN, Hermann, 67269 Grünstadt (DE); KÖRNER, Reinhard, 67227 Frankenthal (DE)
(74) Vertreter: Isenbruck, Günter
(86) Internationale Anmeldenummer: PCT/EP2002/009600
(87) Internationale Veröffentlichungsnummer: WO 2003/020421

(56) Entgegenhaltungen:
- EP-A- 0 729 937
- EP-A- 0 742 045
- EP-A- 0 926 130
- DE-A- 4 343 890

## Beschreibung

Die Erfindung betrifft ein Verfahren zur Herstellung von 3-Aminomethyl-3,5,5-trimethylcyclohexylamin (Isophorondiamin, IPDA) mit einem cis/trans-Isomerenverhältnis von mindestens 70/30 aus 3-Cyano-3,5,5-trimethylcyclohexanon (Isophoronnitril, IPN), H₂ und NH₃, wobei in Gegenwart eines Hydrierkatalysators hydriert wird, dessen Alkalimetallgehalt ≤ 0,03 Gew.-% - berechnet als Alkalimetalloxid - ist. Die Erfindung betrifft ferner ein Verfahren zur Herstellung solcher Katalysatoren sowie die Katalysatoren selbst.

IPDA wird als Ausgangsprodukt zur Herstellung von Isophorondiisocyanat (IPDI), einer Isocyanatkomponente für Polyurethansysteme, als Aminkomponente für Polyamide und als Härter für Epoxidharze verwendet. IPDA wird üblicherweise aus IPN hergestellt, wobei in Gegenwart von Ammoniak, Wasserstoff und üblichen Hydrierkatalysatoren die Carbonylgruppe in eine Aminogruppe und die Nitrilgruppe in eine Aminomethylgruppe überführt werden. Man erhält Gemische aus cis-IPDA und trans-IPDA. Beide Isomere weisen unterschiedliche Reaktivitäten auf, was für die vorgesehene technische Anwendung von Bedeutung ist. Gemäß DE-A 42 11 454 werden durch Verwendung eines IPDA-Isomerengemischs, bestehend aus über 40% des trans-Isomeren und unter 60% des cis-Isomeren als Reaktionskomponente in Polyadditionsharzen, wie insbesondere Epoxidharzen, sowohl die Topfzeit verlängert als auch die maximale Härtungstemperatur erniedrigt. Zur Erzielung einer möglichst hohen Reaktionsgeschwindigkeit werden umgekehrt IPDA-Isomerengemische bevorzugt, welche einen möglichst hohen Anteil an dem cis-Isomeren (≥ 70%) aufweisen. Kommerziell erhältliches IPDA besitzt deshalb ein cis/trans-Isomerenverhältnis von 75/25.

Unterschiedliche Verfahren zur Erzielung eines hohen cis/trans-Verhältnisses sind bereits bekannt:
Gemäß der DE-A 43 43 890 erfolgt die aminierende Hydrierung des IPN zum IPDA, indem man ein Gemisch aus IPN, Ammoniak und einem C₁-C₃-Alkohol in Gegenwart von Wasserstoff über einen mit einem Co- und/oder Ru-Festbettkatalysator ausgestatteten Rieselbettreaktor bei 3 bis 8 MPa und einer Temperatur von 40 bis 150°C, vorzugsweise 90 bis 130°C, rieseln lässt und das Reaktionsgemisch destillativ aufarbeitet. Bei Verwendung eines Ru-Trägerkatalysators werden hohe cis/trans-Verhältnisse von 84/16 (Gesamtausbeute an IPDA: 81%), bei Verwendung eines Co-Trägerkatalysators lediglich cis/trans-Verhältnisse von 60/40 (Gesamtausbeute an IPDA: 87%) erreicht. Durch die Kombination eines Ru- und eines Co-Katalysators gelingt es, IPDA in einem ähnlich hohen cis/trans-Verhältnis wie bei alleiniger Verwendung eines Ru-Katalysators zu erhalten, allerdings in einer höheren Ausbeute als bei alleiniger Verwendung dieses Ru-Katalysators.
Die DE-A 43 43 891 beschreibt ein Verfahren zur Herstellung von IPDA, wobei IPN in Gegenwart von Ammoniak und einem Suspensions- oder Festbett-Hydrierkatalysator aus der Reihe der Co-, Ni- und Edelmetallkatalysatoren mit Wasserstoff bei einem Druck von 3 bis 20 MPa und einer Temperatur bis zu 150°C umgesetzt und das erhaltene Reaktionsgemisch destillativ aufgearbeitet wird, wobei die Besonderheit des Verfahrens darin besteht, die Reaktion zweistufig in genau definierten Temperaturbereichen durchzuführen. Ein cis/trans-Isomerenverhältnis von 80/20 lässt sich in einer Gesamtausbeute an IPDA von 91,9% erzielen.

In dem Verfahren der EP-A 0 926 130 wird die Hydrierung in Gegenwart einer Säure an Katalysatoren durchgeführt, die Kupfer und/oder ein Metall der achten Nebengruppe des Periodensystems enthalten. Es werden sowohl Lewis- als auch Brönstedt-Säuren eingesetzt; bevorzugt wird 2-Ethylhexansäure verwendet. Die cis/trans-Verhältnisse sind im allgemeinen ≥ 70/30 bei einer Gesamtausbeute an IPDA ≥ 90%.

Das Verfahren der EP-B 0 729 937 ist dadurch charakterisiert, dass das Verfahren in drei räumlich voneinander getrennten Reaktionsräumen durchgeführt wird, wobei cobalt-, nickel-, ruthenium- und/oder andere edelmetallhaltige Katalysatoren eingesetzt werden. Vor dem zweiten Reaktor wird wäßrige NaOH-Lösung zudosiert, wodurch die Bildung von ringförmigen Nebenprodukten wie 1,3,3-Trimethyl-6-azabicyclo[3,2,1]octan verringert wird.

Die EP-A 0 742 045 beschreibt Cobalt-Katalysatoren, deren katalytisch aktive Masse aus 55 bis 98 Gew.-% Cobalt, 0,2 bis 15 Gew.-% Phosphor, 0,2 bis 15 Gew.-% Mangan und 0,05 bis 5 Gew.-%, bevorzugt 0,1 bis 3 Gew.-%, besonders bevorzugt 0,13 bis 1 Gew.-%, Alkalimetall, jeweils berechnet als Oxid, besteht. Diese Katalysatoren können in Verfahren zur Hydrierung von organischen Nitrilen und/oder Iminen wie IPN bei Temperaturen von 60 bis 150°C und Drücken von 50 bis 300 bar eingesetzt werden. Angaben über das cis/trans-Verhältnis des so hergestellten IPDA werden nicht gemacht.

Nachteilig an den bekannten Verfahren zur Herstellung von IPDA ist die Bildung von schwer abtrennbaren Nebenprodukten wie HCN-Eliminierungsprodukten, methylierten Nebenprodukten und/oder unvollständig hydrierten Zwischenprodukten (siehe unten).

Aufgabe der Erfindung ist es, ein Verfahren zur Herstellung von Isophorondiamin (IPDA) bereitzustellen, durch das die Nachteile des Standes der Technik vermieden werden. Es sollen zudem ein geeigneter Katalysator und ein Verfahren zur Herstellung dieses Katalysators gefunden werden.

Die Aufgabe wird erfindungsgemäß gelöst durch ein Verfahren zur Herstellung von 3-Aminomethyl-3,5,5-trimethylcyclohexylamin (IPDA) mit einem cis/trans-Isomerenverhältnis von mindestens 70/30 aus 3-Cyano-3,5,5-trimethylcyclohexanon (IPN), NH₃ und H₂, wobei in Gegenwart eines Hydrierkatalysators bei Temperaturen von 50 bis 200°C und einem Druck von 50 bis 300 bar hydriert wird, **dadurch gekennzeichnet, daß** der Alkalimetallgehalt des Hydrierkatalysators ≤ 0,03 Gew.-% - berechnet als Alkalimetalloxid und bezogen auf das Gesamtgewicht des Katalysators - ist. Bevorzugt erfolgt die Hydrierung bei Temperaturen von 60 bis 160°C, besonders bevorzugt bei Temperaturen von 80 bis 130°C, und Drücken von 100 bis 250 bar, besonders bevorzugt bei Drücken von 200 bis 250 bar.

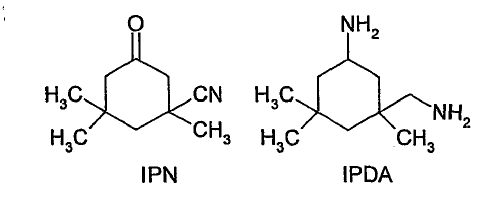

Der Alkalimetallgehalt umfaßt den Gehalt an Li, Na, K, Rb und Cs, insbesondere wird darunter der Gehalt an Na verstanden.

Vergleicht man die Verfahren zur IPDA-Herstellung unter Verwendung alkalimetallhaltiger Katalysatoren mit den erfindungsgemäßen Verfahren unter Verwendung weitgehend alkalimetallfreier Katalysatoren, so stellt man fest, daß die Reaktion bei Verwendung der erfindungsgemäßen weitgehend alkalimetallfreien Katalysatoren selektiver in Bezug auf das gewünschte Produkt IPDA verläuft. Die Bildung von Nebenprodukten beträgt insgesamt ≤ 10 Flächen-%, bevorzugt ≤ 7 Flächen-%, besonders bevorzugt ≤ 5 Flächen-%, wie durch gaschromatographische Analytik des Reaktionsrohprodukts ermittelt wurde. Es findet weniger HCN-Eliminierung statt (Nebenprodukte IIa und IIb); zudem werden kaum methylierte Nebenprodukte (IIIa, IIIb) gebildet. Dies ist besonders vorteilhaft, da diese nur schwer vom gewünschten Produkt IPDA abtrennbar sind. Auch die Menge an unvollständig hydriertem Nebenprodukt Aminonitril (IV), welches destillativ kaum von IPDA abgetrennt werden kann, nimmt ab. Zwar wird mehr cyclisches Nebenprodukt 1,3,3-Trimethyl-6-azabicyclo[3,2,1]octan (V) gebildet, dieses ist jedoch von IPDA leicht destillativ abtrennbar.

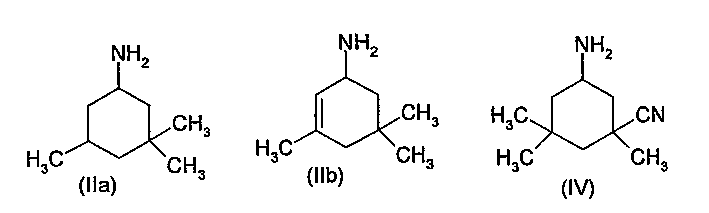

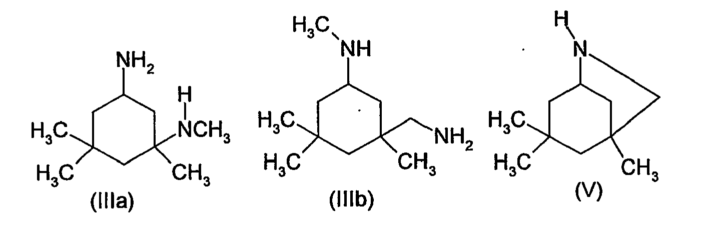

Durch das erfindungsgemäße Verfahren lassen sich cis/trans-Verhältnisse von mindestens 70/30, bevorzugt von mindestens 73/27, besonders bevorzugt von mindestens 75/25 erzielen. Das cis/trans-Verhältnis lässt sich beispielsweise bestimmen durch gaschromatographische (GC) Untersuchung des Reaktionsrohprodukts. Hierbei werden die ermittelten Flächen der Peaks von cis- und trans-IPDA ins Verhältnis zueinander gesetzt.

Die Gesamtausbeute an IPDA beträgt hierbei im allgemeinen ≥ 90%, insbesondere ≥ 93%, vor allem insbesondere ≥ 95%. Die Reinheit beträgt im allgemeinen mindestens 98%, insbesondere mindestens 99%, vor allem insbesondere von 99,3 bis 99,7% und läßt sich durch GC-Analytik des Reaktionsrohprodukts bestimmen.

Bei der Umsetzung von IPN zu IPDA wird 3-Cyano-3,5,5-trimethyl-cyclohexanonimin (I) als Zwischenprodukt gebildet. Diese Iminierung wird insbesondere als separate Iminierungsstufe vor der Hydrierung bei Temperaturen von 20 bis 150°C, bevorzugt von 30 bis 130°C, besonders bevorzugt von 50 bis 100°C, und Drücken von 50 bis 300 bar, bevorzugt von 100 bis 250 bar, besonders bevorzugt von 200 bis 250 bar, durchgeführt. Die anschließende Hydrierung kann dann ein- oder zweistufig erfolgen (siehe unten).

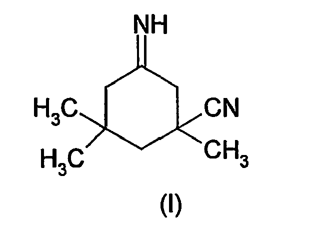

Bevorzugt wird das erfindungsgemäße Verfahren zweistufig (eine Iminierungs- und eine Hydrierstufe) mit folgenden Schritten durchgeführt:
A) Umsetzung von IPN, NH₃ und H₂ bei Temperaturen von 20 bis 150°C und einem Druck von 50 bis 300 bar, gegebenenfalls in Gegenwart eines Iminierungskatalysators;
B) Umsetzung des in Schritt A) erhaltenen Reaktionsgemischs in Gegenwart eines Hydrierkatalysators bei Temperaturen von 60 bis 100°C und einem Druck von 50 bis 300 bar;
wobei Schritt A) und Schritt B) entweder im gleichen Reaktionsraum oder in zwei räumlich voneinander getrennten Reaktionsräumen durchgeführt werden.

Bevorzugt erfolgt die Umsetzung gemäß Schritt A) bei Temperaturen von 30 bis 130°C, besonders bevorzugt bei Temperaturen von 50 bis 100°C, und bevorzugten Drücken von 100 bis 250 bar. Schritt A) läßt sich sowohl ohne als auch mit Katalysator durchführen,
wobei bei Einsatz eines Katalysators die Reaktionsgeschwindigkeit erhöht ist.

In Schritt B) betragen die Drücke bevorzugt von 100 bis 250 bar. Die eingesetzten Katalysatoren werden weiter unten detailliert beschrieben.

Besonders bevorzugt wird das erfindungsgemäße Verfahren in drei räumlich voneinander getrennten Reaktionsräumen enthaltend folgende Reaktionsschritte (ein Iminierungsschritt und zwei Hydrierschritte) durchgeführt:
a) Umsetzung von IPN in einem ersten Reaktionsraum mit überschüssigem Ammoniak bei Temperaturen von 20 bis 150°C und Drücken von 50 bis 300 bar im wesentlichen zu 3-Cyano-3,5,5-trimethyl-cyclohexanonimin (I), gegebenenfalls an einem aciden Metalloxidkatalysator,
b) Hydrierung der in Schritt a) erhaltenen Reaktionsprodukte in einem zweiten Reaktionsraum mit Wasserstoff in Gegenwart von überschüssigem Ammoniak an einem Hydrierkatalysator bei Temperaturen von 60 bis 100°C und Drücken von 50 bis 300 bar, und
c) Hydrierung der in Schritt b) erhaltenen Reaktionsprodukte in einem dritten Reaktionsraum in Gegenwart von Wasserstoff und Ammoniak an einem Hydrierkatalysator bei Temperaturen von 110 bis 160°C und Drückern von 50 bis 300 bar.

Dieses dreistufige Verfahren wird im folgenden detailliert beschrieben:

### Schritt a)

In einer ersten Verfahrensstufe setzt man IPN mit überschüssigem Ammoniak in einem ersten Reaktionsraum bei Temperaturen von 20 bis 150°C, vorzugsweise von 30 bis 130°C, besonders bevorzugt von 50 bis 100°C, und Drücken von 50 bis 300 bar, vorzugsweise von 100 bis 250 bar, im wesentlichen zu 3-Cyano-3,5,5-trimethylcyclohexanonimin (I) um.

Schritt a) läßt sich sowohl ohne als auch mit Katalysator durchführen, wobei bei Einsatz eines Katalysators die Reaktionsgeschwindigkeit erhöht ist. Als acide Metalloxidkatalysatoren eignen sich Aluminiumoxid, Siliciumdioxid, Titandioxid, Zirkondioxid, Aktivkohle sowie deren Mischungen. Bevorzugt verwendet man Aluminiumoxid, Titandioxid, Zirkondioxid, sowie deren Mischungen, insbesondere Aluminiumoxid und/oder Titandioxid.

Bei der Iminierung hält man eine Katalysatorbelastung von 0,01 bis 10, vorzugsweise von 0,05 bis 7, besonders bevorzugt von 0,1 bis 5, kg IPN pro kg Katalysator und Stunde ein. Pro mol IPN setzt man bei der Iminierung zweckmäßig 5 bis 50 mol, bevorzugt 10 bis 40 mol, besonders bevorzugt 20 bis 30 mol, NH₃ ein. Die Iminierung des IPN kann in Anwesenheit eines Lösungsmittels, wie z.B. Alkanolen oder Tetrahydrofuran durchgeführt werden, man kann aber auch ohne Zusatz eines Lösungsmittels arbeiten.

Die Iminierung wird bevorzugt kontinuierlich durchgeführt, z.B. in Druckbehältern oder Druckbehälterkaskaden. Nach einer besonders bevorzugten Ausführungsform werden IPN und NH₃ in Sumpf- oder Rieselfahrweise durch einen Reaktor geleitet, in dem der Iminierungskatalysator in Form eines festen Bettes angeordnet ist, oder sie werden in einem Schachtofen miteinander umgesetzt.

### Schritt b)

Die in Schritt a) erhaltenen Reaktionsprodukte werden in einem zweiten Reaktionsraum mit 3 bis 10000, bevorzugt 4,5 bis 100, Moläquivalent Wasserstoff - gegebenenfalls nach Zufuhr von weiterem Ammoniak - einer katalytischen Hydrierung unterzogen.

Bei der Hydrierung hält man eine Temperatur von 60 bis 100°C und einen Druck von 50 bis 300 bar, bevorzugt von 100 bis 250 bar, ein.

Die Katalysatorbelastungen liegen zweckmäßig im Bereich von 0,01 bis 5 kg Verbindung (I) pro 1 Katalysator und Stunde [kg/l•h], bevorzugt bei 0,02 bis 2,5 kg Verbindung (I) pro 1 Katalysator und Stunde, besonders bevorzugt bei 0,05 bis 2 kg Verbindung (I) pro 1 Katalysator und Stunde.

Die Hydrierung wird vorzugsweise in flüssigem Ammoniak durchgeführt. Pro mol 3-Cyano-3,5,5-trimethyl-cyclohexanonimin (I) setzt man 5 bis 500 mol, bevorzugt 10 bis 400 mol, besonders bevorzugt 20 bis 300 mol, NH₃ ein. Zweckmäßigerweise wählt man mindestens das NH₃-Angebot, das bei der vorgelagerten Herstellung der Verbindung I aus IPN eingestellt wurde. Der NH₃-Anteil kann jedoch auch vor der Hydrierung durch Zugabe von zusätzlichem NH₃ auf den gewünschten Wert erhöht werden.

Die aminierende Hydrierung der in Schritt a) erhaltenen Reaktionsprodukte führt man bevorzugt kontinuierlich z.B. in druckfesten Rührbehältern oder in einer Rührbehälterkaskade durch. In einer besonders bevorzugten Ausführungsform werden Reaktoren eingesetzt, in denen das Produktgemisch aus der Iminierung in Sumpf- oder Rieselfahrweise über ein fest angeordnetes Katalysatorbett geleitet wird.

Der Reaktoraustrag enthält gegebenenfalls noch nicht vollständig umgesetzte Komponenten, wie z. B. das Aminonitril (IV), welches von IPDA destillativ kaum abtrennbar ist.

### Schritt c)

Der aus b) erhaltene Reaktoraustrag wird in einer dritten Stufe in Gegenwart von Wasserstoff und Ammoniak bei 110 bis 160°C und 50 bis 300 bar, bevorzugt 100 bis 250 bar, umgesetzt. Hierbei kann sowohl der gleiche als auch ein anderer Katalysator wie in Schritt b) eingesetzt werden. Zweckmäßigerweise wählt man Ammoniak und Wasserstoffangebot, wie es sich im Reaktoraustritt der Stufe b) einstellt.

In einer bevorzugten Ausführungsform des erfindungsgemäßen Verfahrens ist der in Schritt c) eingesetzte Reaktor deutlich kleiner als der in Schritt b) eingesetzte Reaktor. Beispielsweise besitzt der in Schritt c) eingesetzte Reaktor ein Leervolumen, das 20 bis 40% des Leervolumens des in Schritt b) eingesetzten Reaktors entspricht.

Nach der Hydrierung wird überschüssiger Ammoniak und gegebenenfalls Wasserstoff, gegebenenfalls unter Druck, abgetrennt. Das so erhaltene Roh-IPDA wird durch fraktionierende Destillation gereinigt.

In den erfindungsgemäßen Verfahren zur Herstellung von IPDA aus IPN können als Hydrierkatalysatoren prinzipiell alle gängigen Hydrierkatalysatoren mit einem Alkalimetallgehalt ≤ 0,03 Gew.-% - berechnet als Alkalimetalloxid und bezogen auf das Gesamtgewicht des Katalysators - eingesetzt werden, die mindestens ein Übergangsmetall ausgewählt aus der Gruppe Cobalt, Ruthenium, Nickel, Eisen, Rhodium, Palladium, Osmium, Iridium, Platin und Kupfer enthalten. Bevorzugt verwendet man Ruthenium- und Cobalt-Katalysatoren sowie deren Mischungen mit einem Alkalimetallgehalt ≤ 0,03 Gew.-% - berechnet als Alkalimetalloxid und bezogen auf das Gesamtgewicht des Katalysators. Besonders bevorzugt sind Cobalt-Katalysatoren mit einem Alkalimetallgehalt ≤ 0,03 Gew.-% - berechnet als Alkalimetalloxid und bezogen auf das Gesamtgewicht des Katalysators. Diese Cobalt-Katalysatoren können darüber hinaus auch in einer Menge von 0,01 bis 10 Gew.-%, bevorzugt von 0,05 bis 5 Gew.-%, besonders bevorzugt von 0,5 bis 3 Gew.-%, - bezogen auf das Gesamtgewicht des Katalysators - mit Übergangsmetallen wie Ru, Ir, Pd, Pt und/oder Ni dotiert sein.

Ganz besonders bevorzugt im erfindungsgemäßen Verfahren sind Cobalt-Katalysatoren, die 55 bis 98 Gew.-%, bevorzugt 75 bis 95 Gew.-%, besonders bevorzugt 85 bis 95 Gew.-%, Cobalt, 0,2 bis 15 Gew.-%, bevorzugt 0,5 bis 10 Gew.-%, besonders bevorzugt 1 bis 6 Gew.-%, Phosphor, 0,2 bis 15 Gew.-%, bevorzugt 2 bis 10 Gew.-%, besonders bevorzugt 3 bis 8 Gew.-%, Mangan und ≤ 0,03 Gew.-% Alkalimetall, jeweils berechnet als Oxid und bezogen auf das Gesamtgewicht des Katalysators, enthalten. Gegebenenfalls enthalten diese Cobalt-Katalysatoren auch einen oder mehrere Promotoren ausgewählt aus der Gruppe Ruthenium, Palladium und Nickel, bevorzugt Ruthenium und/oder Nickel, besonders bevorzugt Ruthenium, in einer Menge von 0,01 bis 10 Gew.-%, bevorzugt von 0,05 bis 5 Gew.-%, besonders bevorzugt von 0,5 bis 3 Gew.-%, jeweils berechnet als Oxid und bezogen auf das Gesamtgewicht des Katalysators.

Der Alkalimetallgehalt der genannten Katalysatoren ist bevorzugt ≤ 0,015 Gew.-%, besonders bevorzugt ≤ 0,01 Gew.%, - berechnet als Alkalimetalloxid und bezogen auf das Gesamtgewicht des Katalysators. Insbesondere ist der Natriumgehalt der genannten Katalysatoren ≤ 0,03 Gew.-%, bevorzugt ≤ 0,015 Gew.-%, besonders bevorzugt ≤ 0,01 Gew.-%, - berechnet als Natriumoxid und bezogen auf das Gesamtgewicht des Katalysators. Diese Katalysatoren mit einem Alkalimetallgehalt ≤ 0,03 Gew.-%, insbesondere einem Natriumgehalt ≤ 0,03 Gew.-%, - berechnet als Alkalimetalloxid und bezogen auf das Gesamtgewicht des Katalysators - sind erfmdungsgemäß. Der Alkalimetall- bzw. Alkalimetalloxid-Gehalt lässt sich durch Atomabsorptionsspektroskopie bestimmen. Die untere analytische Nachweisgrenze für Alkalimetalle, insbesondere Na, liegt bei diesem Verfahren bei 0,003 Gew.-%.

Beim dreistufigen Verfahren mit den Schritten a), b) und c) muß nur einer der beiden in den Schritten b) und c) eingesetzten Hydrierungskatalysatoren einen Alkalimetallgehalt ≤ 0,03 Gew.-% - berechnet als Alkalimetalloxid und bezogen auf das Gesamtgewicht des Katalysators - aufweisen. Bei dem anderen Katalysator ist dieses Merkmal optional.

Gegenstand der vorliegenden Erfindung ist ebenfalls ein Verfahren zur Herstellung eines Hydrierkatalysators mit einem Alkalimetallgehalt ≤ 0,03 Gew.-% - berechnet als Alkalimetalloxid und bezogen auf das Gesamtgewicht des Katalysators, der mindestens ein Übergangsmetall ausgewählt aus der Gruppe Co, Ru, Ni, Fe, Rh, Pd, Os, Ir, Pt und Cu enthält, enthaltend folgende Schritte:
i) Fällen mindestens eines der obengenannten Übergangsmetalle in Form seines Carbonats, Hydroxids und/oder Oxids aus einer wäßrigen Lösung enthaltend mindestens ein wasserlösliches Salz eines der genannten Übergangsmetalle mit einer wäßrigen Lösung enthaltend mindestens eine Substanz ausgewählt aus der Gruppe Ammoniumcarbonat, Ammoniumhydrogencarbonat, Ammoniumcarbamat, Ammoniumoxalat, Ammoniummalonat, Ammoniak und Urotropin unter Rühren - gegebenenfalls unter Zusatz von Promotoren in Form ihrer wasserlöslichen Verbindungen;
ii) Abtrennen des in Schritt i) erhaltenen Niederschlags;
iiia) Trocknen des so erhaltenen Niederschlags bei Temperaturen von 50 bis 200°C und Vermahlen zu Katalysatorpulver, oder
iiib) Aufschlämmen des so erhaltenen Niederschlags und Versprühen der so erhaltenen Suspension bei Temperaturen von 100 bis 600°C zu einem Katalysatorsprühpulver - gegebenenfalls unter Zusatz von Promotoren in Form ihrer Salze;
iv) Calcinieren der in Schritt iiia) oder iiib) erzeugten Katalysatorpulver bei Temperaturen von 300 bis 1000°C und Verformung zu Katalysatorformkörpern, wobei gegebenenfalls vor und/oder während und/oder nach der Verformung zu Katalysatorformkörpern Promotoren in Form ihrer Salze zugegeben werden;
v) Trocknen und Calcinieren der in Schritt iv) erzeugten Katalysatorformkörper;
vi) Reduktion der getrockneten und calcinierten Katalysatorformkörper in einer H₂/N₂-Atmosphäre bei erhöhter Temperatur, wobei die Zusammensetzung der H₂/N₂-Atmosphäre und die Temperatur variiert wird;
vii) gegebenenfalls Passivieren der reduzierten Katalysatorformkörper bei Temperaturen von 20 bis 60°C, wobei gegebenenfalls anschließend Promotoren in Form ihrer Salze auf die Katalysatorformkörper aufgebracht werden.

Durch das vorstehend genannte Verfahren werden insbesondere Hydrierkatalysatoren mit einem Natriumgehalt ≤ 0,03 Gew.-% erhalten. Das Verfahren wird nun detailliert erläutert, so wird z. B. der Begriff "erhöhte Temperatur" (Schritt vi)) definiert. Unter Raumtemperatur versteht man im Rahmen dieses Verfahrens Temperaturen von 15 bis 35°C, insbesondere Temperaturen von 20 bis 30°C.

### Schritt i)

Aus einer wäßrigen Lösung enthaltend mindestens ein wasserlösliches Salz eines Übergangsmetalls ausgewählt aus der Gruppe Co, Ru, Ni, Fe, Rh, Pd, Os, Ir, Pt und Cu, bevorzugt mindestens ein wasserlösliches anorganisches Salz eines dieser Übergangsmetalle, wird durch Zugabe einer wäßrigen Lösung (Fällösung) enthaltend mindestens eine Substanz (Fällreagenz) ausgewählt aus der Gruppe Ammoniumcarbonat, Ammoniumhydrogencarbonat, Ammoniumcarbamat, Ammoniumoxalat, Ammoniummalonat, Ammoniak und Urotropin unter Rühren mindestens eines der oben genannten Übergangsmetalle in Form seines Carbonats, Hydroxids und/oder Oxids bei Temperaturen von 30 bis 90°C, bevorzugt bei Temperaturen von 40 bis 80°C, besonders bevorzugt bei Temperaturen von 45 bis 55°C gefällt. Wird mit Ammoniumcarbonat oder Ammoniumhydrogencarbonat als Fällreagenz gearbeitet, so arbeitet man im allgemeinen bei einem pH-Wert von 5,5 bis 9,0, bevorzugt bei einem pH-Wert von 6,0 bis 8,0, besonders bevorzugt bei einem pH-Wert von 6,2 bis 6,8. Wird mit Ammoniumcarbamat, Ammoniumoxalat, Ammoniummalonat, Ammoniak und/oder Urotropin gefällt, so liegt der pH-Wert der Fällösung im allgemeinen ≤ 5, bevorzugt ≤ 2, besonders bevorzugt ≤ 1,5. Von den angegebenen Übergangsinetallsalzen werden bevorzugt Co- und Ru-, besonders bevorzugt Co-Salze, eingesetzt. Von den Fällreagenzien wird bevorzugt Ammoniumcarbonat verwendet. Die Übergangsmetallsalzlösung enthält gegebenenfalls gewünschte Promotoren wie Mangan, Phosphor und/oder Ruthenium in Form ihrer wasserlöslichen Verbindungen.

Die Konzentrationen sowohl der Übergangsmetallsalzlösung als auch der Fällösung sollten so eingestellt werden, dass die resultierende Fällmaische noch gerührt werden kann. Werden die Promotoren nicht in diesem Schritt mitgefällt, können sie in Schritt iiib), Schritt iv) und/oder Schritt vii) zugeführt werden. Die Zugabe der wäßrigen Fällösung wird so lange fortgesetzt, bis eine vollständige Ausfällung erreicht ist. Der entstandene Niederschlag kann erforderlichenfalls nachgerührt werden.

### Schritt ii)

Anschließend wird der in Schritt i) erhaltene Niederschlag mit üblichen technischen Mitteln abgetrennt und gegebenenfalls von unerwünschten wasserlöslichen Ionen wie z. B. Nitraten freigewaschen.

### Schritt iiia)

Der so erhaltene Niederschlag kann dann bei Temperaturen von 50 bis 200°C getrocknet und anschließend zu Katalysatorpulver vermahlen werden.

### Schritt iiib)

Alternativ zu Schritt iiia) ist ein Aufschlämmen des so erhaltenen Niederschlags und ein anschließendes Versprühen der entstandenen Maische (Suspension) in einem Sprühturm bei Temperaturen zwischen 100 und 600°C möglich. Hierbei entsteht ein Katalysatorsprühpulver.

Wird das Versprühen (Schritt iiib)) gewählt, so können dem Katalysator auch in diesem Verfahrensschritt Promotoren wie Mangan, Ruthenium und/oder Phosphor in Form ihrer Salze beigefügt werden.

### Schritt iv)

Die in Schritt iiia) oder Schritt iiib) erzeugten Katalysatorpulver können calciniert werden. Die Calcinierung wird in einem Schritt bei Endtemperaturen von 300 bis 1000°C, bevorzugt bei Endtemperaturen von 400 bis 800°C, besonders bevorzugt bei Endtemperaturen von 500 bis 600°C, durchgeführt.

Die calcinierten Katalysatorpulver können in verschiedener Weise zu Katalysatorformkörpern verformt werden. So ist es möglich, die Pulver zu tablettieren, zu extrudieren oder mit Hilfe einer Strangpresse zu Strängen bestimmter Form und Größe zu verpressen. Als Formen sind alle geometrischen Körper herstellbar, die sich in Festbettreaktoren einfüllen lassen. In allen Fällen können dem Pulver Verformungshilfsmittel wie Graphit oder Stearinsäure beigemischt werden.

Sowohl vor als auch nach und/oder während der Verformung zu Katalysatorformkörpern können Promotoren in Form ihrer Salze zugegeben werden.

### Schritt v)

Die so erzeugten Katalysatorformkörper werden anschließend bei Temperaturen von 70 bis 200°C, bevorzugt bei Temperaturen von 90 bis 150°C, besonders bevorzugt bei Temperaturen von 100 bis 130°C, getrocknet und danach calciniert. Die Calcinierung wird in einem Schritt bei Endtemperaturen von 300 bis 1000°C, bevorzugt bei Endtemperaturen von 700 bis 1000°C, besonders bevorzugt bei Endtemperaturen von 800 bis 950°C, durchgeführt.

### Schritt vi)

Zur Reduktion werden die getrockneten und calcinierten Katalysatorformkörper bei Raumtemperatur mit Stickstoff gespült und es wird dann unter Stickstoffatmosphäre ein Druck von 2 bis 10 bar, bevorzugt 4 bis 8 bar, eingestellt.

Anschließend wird eine H₂-Menge zugegeben, welche in der Regel 30 bis 70% des Stickstoffstroms, bevorzugt 40 bis 60% des Stickstoffstroms, entspricht. Die Temperatur wird dann in der Regel innerhalb von 2 bis 24 h, bevorzugt innerhalb von 5 bis 15 h, von Raumtemperatur auf 200 bis 400°C, bevorzugt auf 250 bis 350°C, besonders bevorzugt auf 280 bis 320°C, erhöht. Diese Endtemperatur wird in der Regel so lange gehalten, bis der gewünschte Reduktionsgrad erreicht ist, der mit Hilfe des aus dem Katalysatorformkörper austretenden Reduktionswasser bestimmbar ist. Anschließend läßt man den reduzierten Katalysatorformkörper im Stickstoffstrom auf Raumtemperatur abkühlen.

### Schritt vii)

Dieser Schritt ist optional. Zur Passivierung, d. h. oberflächlichen Anoxidation, des reduzierten Katalysatorformkörpers dosiert man dem Stickstoffstrom sukzessive Luft zu, und zwar so langsam, dass die Temperatur im Katalysatorbett den Wert von 60°C nicht überschreitet, die Temperatur also 20 bis 60°C, bevorzugt 20 bis 50°C, besonders bevorzugt 20 bis 40°C, beträgt. Der Austausch von Stickstoff gegen Luft wird so lange fortgeführt, bis der den Katalysatorformkörper durchströmende Gasstrom aus 100% Luft besteht.

Setzt man in dem vorstehend erläuterten Verfahren beispielsweise Cobaltsalze ein, so erhält man Cobalt-Katalysatoren mit einer spezifischen Oberfläche von größer oder gleich 12 m²/g, also 12 bis 500 m²/g, bevorzugt 15 bis 200 m²/g, besonders bevorzugt 18 bis 100 m²/g und einer Porosität von ≥ 0,10 cm³/g, also 0,10 bis 1,00 cm³/g, bevorzugt 0,11 bis 0,40 cm³/g, besonders bevorzugt 0,12 bis 0,20 cm³/g.

Es lassen sich so auch gemischte Ru-/Co-Katalysatoren herstellen, in denen der Anteil des Ru von 0,01 bis 10 Gew.-%, bevorzugt von 0,1 bis 5 Gew.-%, besonders bevorzugt von 0,5 bis 2 Gew.-%, - jeweils berechnet als Oxid - beträgt. Ein solcher Co-Katalysator wird hierbei entweder durch gemeinsames Fällen von Co- und Ru-Salzen in Schritt i) des Verfahrens oder durch Zugabe von Rutheniumsalzen in den Schritten iiib), iv) und/oder vi) hergestellt.

Die nach dem vorstehend genannten Verfahren herstellbaren Katalysatoren mit einem Alkalimetallgehalt ≤ 0,03 Gew.-%, insbesondere einem Natriumgehalt ≤ 0,03 Gew.-%, - berechnet als Alkalimetalloxid/Natriumoxid - werden in den erfindungsgemäßen Verfahren zur Herstellung von IPDA aus IPN als Vollkontakte eingesetzt. Es lassen sich jedoch auch - nach anderen nicht-erfindungsgemäßen Herstellverfahren gewonnene - Katalysatoren mit einem Alkalimetallgehalt ≤ 0,03 Gew.-%, insbesondere einem Natriumgehalt ≤ 0,03 Gew.-%, - berechnet als Alkalimetalloxid/Natriumoxid - in diesen Verfahren zur Herstellung von IPDA aus IPN einsetzen.

Die Erfindung wird nun in den nachfolgenden Ausführungsbeispielen zusätzlich näher erläutert.

### Ausführungsbeispiel 1:

### Herstellung eines weitgehend Na-freien Co-Katalysators

Ausgehend von einer Lösung, die 10 Gew.-% Cobalt, 0,55 Gew.-% Mangan und 0,45 Gew.-% H₃PO₄ enthält, wurde gemäß dem obigen Verfahren ein weitgehend Na-freier Co-Katalysator hergestellt.

Tabelle 1 gibt die Zusammensetzung des Katalysators wieder, wie sie durch Elementaranalytik (Atomabsorptionsspektroskopie) bestimmt wurde. Der Katalysator besitzt eine spezifische Oberfläche von 16 m²/g und eine Porosität von 0,12 cm³/g.

**Tabelle 1**

| | Als Element | Als Oxid |
|---|---|---|
| Co[Gew.-%] | 76 | 87,9 |
| Mn [Gew.-%] | 4,2 | 6,6 |
| P [Gew.-%] | 1,8 | 5,5 |
| Na [Gew.-%] | <0,01 | <0,01 |
| Oxidisch gebundener Sauer-stoff | Bilanz zu 100 Gew.-% | |

### Vergleichsbeispiel 1

Als Vergleichskatalysator wurde ein Na-haltiger Katalysator entsprechend dem Katalysator A der EP-A 0 742 045 hergestellt.

### Ausführungsbeispiel 2: Herstellung von IPDA

Die aminierende Hydrierung von IPN zu IPDA erfolgt in einem kontinuierlichen Prozeß in drei hintereinander geschalteten Reaktoren bei einem Druck von 250 bar. Der weitgehend Na-freie (erfindungsgemäße) bzw. Na-haltige Cobalt-Katalysator wird unter Wasserstoffatmosphäre mit einer Aufheizrate von 2°C/min auf bis zu 280°C hochgeheizt. Nach 12 h bei dieser Temperatur wird auf die jeweilige Reaktionstemperatur zurückgefahren.

Durch den ersten Reaktor (Iminierungsreaktor 200 ml) gefüllt mit γ-Al₂O₃ als Katalysator, fährt man in Sumpffahrweise Isophoronnitril (130ml/h), Ammoniak (600 g/h) und 300 l/h Wasserstoff bei ca. 80-100 °C. Dort erfolgt die Iminierung. Das Reaktionsgemisch wird in den ersten Reaktor (Rieselbettreaktor) mit dem Cobalt-Katalysator gefahren. Dort ist die Temperatur 90°C. Im dritten (letzten) Reaktor erfolgt die Nachhydrierung in Gegenwart des Cobalt-Katalysators in Sumpffahrweise bei 130°C. Das Produktgemisch wird entspannt und abgekühlt und gaschromatographisch analysiert.

Ergebnis beider Katalysatoren im Vergleich:

| | Na-haltiger Co-Katalysator (Vergleichsbeispiel 1) | Na-freier Co-Katalysator (erfindungsgemäß) |
|---|---|---|
| Ausbeute gesamt | 92,9 | 92,5 |
| cis/trans-Verhältnis | 68,7 | 75,8 |
| HCN-Abspaltungsprodukte (**IIa** und **IIb**) | 4,6 | 1,5 |
| methylierte Nebenprodukte (**IIIa** und **IIIb**) | 0,7 | 0,1 |
| ringförmiges Nebenprodukt (**V**) | 0,4 | 2,6 |
| Aminonitril (**IV**) | 0,2 | 0,1 |

Bei einem Vergleich der Ergebnisse fällt auf, daß durch Verwendung eines erfmdungsgemäßen weitgehend Na-freien Katalysators IPDA etwa in derselben Gesamtausbeute erhalten wird wie bei Verwendung eines Na-haltigen Katalysators. Durch Verwendung des erfindungsgemäßen Katalysators wird jedoch ein höheres cis/trans-Verhältnis erzielt. Zudem sind die Mengen an schwer abtrennbaren Nebenprodukten (IIa, IIb, IIIa, IIIb und IV) geringer, während die Menge an leicht abtrennbarem Nebenprodukt (V) geringfügig erhöht ist.

## Patentansprüche

1. Verfahren zur Herstellung von 3-Aminomethyl-3,5,5-trimethylcyclohexylamin (Isophorondiamin, IPDA) mit einem cis/trans-Isomerenverhältnis von mindestens 70/30 aus 3-Cyano-3,5,5-trimethylcyclohexanon (Isophoronnitril, IPN), NH₂, und H₂, wobei in Gegenwart eines 55 bis 98 Gew.-% Cobalt 0,2 bis 15 Gew.-% Phosphor und 0,2 bis 15 Gew.-% Mangan sowie gegebenenfalls Ruthenium, jeweils berechnet als Oxid und bezogen auf das Gesamtgewicht des Katalysators, enthaltenden Hydrierkatalysators bei Temperaturen von 50 bis 200°C und einem Druck von 50 bis 300 bar hydriert wird, **dadurch gekennzeichnet, daß** der Alkalimetallgehalt des Hydrierkatalysators ≤ 0,03 Gew.-% - berechnet als Alkalimstalloxid und bezogen auf das Gesamtgewicht des Katalysators - ist.

2. Verfahren nach Anspruch 1, **dadurch gekennzeichnet, daß** das Verfahren zweistufig mit folgenden Schritten durchgeführt wird:
A) Umsetzung von IPN, NH₂ und H₂ bei Temperaturen von 20 bis 150°C und einem Druck von 50 bis 300 bar, gegebenenfalls in Gegenwart eines Iminierungskatalysators;
B) Umsetzung des in Schritt A) erhaltenen Reaktionsgemischs in Gegenwart eines Hydrierkatalysators bei Temperaturen von 60 bis 100°C und einem Druck von 50 bis 300 bar;
wobei Schritt A) und Schritt B) entweder im gleichen Reaktionsraum oder in zwei räumlich voneinander getrennten Reaktionsräumen durchgeführt werden.

3. Verfahren nach Anspruch 1, **dadurch gekennzeichnet, daß** das Verfahren in drei räumlich voneinander getrennten Reaktionsräumen mit folgenden Reaktionsschritten durchgeführt wird:
a) Umsetzung von IPN in einem ersten Reaktionsraum mit überschüssigem Ammoniak bei Temperaturen von 20 bis 150°C und Drücken von 50 bis 300 bar im wesentlichen zu 3-Cyano-3,5,5-trimethylcyclohexanonimin, gegebenenfalls an einem aciden Metalloxidkatalysator,
b) Hydrierung der in Schritt a) erhaltenen Reaktionsprodukte in einem zweiten Reaktionsraum mit Wasserstoff In Gegenwart von überschüsslgem Ammoniak an einem Hydrierkatalysator bei Temperaturen von 60 bis 100°C und Drücken von 50 bis 300 bar, und
c) Hydrierung der in Schritt b) erhaltenen Reaktionsprodukte in einem dritten Reaktionsraum in Gegenwart von Wasserstoff und Ammoniak an einem Hydrierkatalysator bei Temperaturen von 110 bis 160°C und Drücken von 50 bis 300 bar.

4. Verfahren nach Anspruch 3, **dadurch gekennzeichnet, dass** Schritt a) bei Temperaturen von 30 bis 130°C und/oder Drücken von 100 bis 250 bar durchgeführt wird.

5. Verfahren nach Anspruch 3 oder 4, **dadurch gekennzeichnet, dass** Schritt b) und/oder Schritt c) bei Drücken von 100 bis 250 bar durchgeführt wird.

6. Verfahren nach einem der Ansprüche 1 bis 5, **dadurch gekennzeichnet, dass** der Alkalimetallgehalt eines Hydrierkatalysators s 0,015 Gew.-%, bevorzugt ≤ 0,01 Gew.-%, - berechnet als Alkalimetalloxid und bezogen auf das Gesamtgewicht des Katalysators - ist.

7. Hydrierkatalysator, wie er in einem der Ansprüche 1 oder 6 definiert ist.

8. Verwendung eines Hydrierkatalysators nach Anspruch 7 zur aminierenden Hydrierung von Isophoronnitril.

9. Verfahren zur Herstellung eines Hydrierkatalysators nach Anspruch 7, enthaltend folgende Schritte:
i) Fällen mindestens eines der obengenannten Übergangsmetalle in Form seines Carbonats, Hydroxids und/oder Oxids aus einer wäßrigen Lösung enthaltend mindestens ein wasserlösliches Salz eines der genannten Übergangsmetalle mit einer wäßrigen Lösung enthaltend mindestens eine Substanz ausgewählt aus der Gruppe Ammoniumcarbonat, Ammoniumhydrogencarbonat, Ammoniumcarbamat, Ammoniumoxalat, Ammoniummalonat, Ammoniak und Urotropin unter Rühren - gegebenenfalls unter Zusatz von Promotoren in Form ihrer wasserlöslichen Verbindungen;
ii) Abtrennen des in Schritt i) erhaltenen Niederschlags;
iiia) Trocknen des so erhaltenen Niederschlags bei Temperaturen von 50 bis 200°C und Vermahlen zu Katalysatorpulver, oder
iiib) Aufschlämmen des so erhaltenen Niederschlags und Versprühen der so erhaltenen Suspension bei Temperaturen von 100 bis 600°C zu einem Katalysatorsprühpulver - gegebenenfalls unter Zusatz von Promotoren in Form ihrer Salze;
iv) Calcinieren der in Schritt iiia) oder iiib) erzeugten Katalysatorpulver bei Temperaturen von 300 bis 1000°C und Verformung zu Katalysatorformkörpem, wobei gegebenenfalls vor oder während oder nach der Verformung zur Katalysatorformkörpern Promotoren in Form ihrer Salze zugegeben werden;
v) Trocknen und Calcinieren der in Schritt iv) erzeugten Katalysatorformkörper;
vi) Reduktion der getrockneten und calcinierten Katalysatorformkörper in einer H₂-/N₂-Atmosphäre bei erhöhter Temperatur, wobei die Zusammensetzung der H₂-/N₂-Atmosphäre und die Temperatur variiert wird;
vii) gegebenenfalls Passivieren der reduzierten Katalysatorformkörper bei Temperaturen von 20 bis 60°C, wobei gegebenenfalls anschließend Promotoren in Form ihrer Salze auf die Katalysatorformkörper aufgebracht werden.

10. Verfahren nach Anspruch 9, **dadurch gekennzeichnet, dass** in Schritt i) mit einer wäßrigen Lösung enthaltend Ammoniumcarbonat gefällt wird.

11. Verfahren nach Anspruch 9 oder 10, **dadurch gekennzeichnet, dass** in Schritt i) eines oder mehrere wasserlösliche Co-Salze eingesetzt werden.

12. Verfahren nach einem der Ansprüche 9 bis 11, **dadurch gekennzeichnet, dass** in Schritt i) als Promotoren ein oder mehrere wasserlösliche Mn-Salze sowie eine oder mehrere wasserlösliche Phosphorverbindungen eingesetzt werden.

## Claims

1. A process for preparing 3-aminomethyl-3,5,5-trimethylcyclohexylamine (isophoronediamine, IPDA) having a cis/trans isomer ratio of at least 70/30 from 3-cyano-3,5,5-trimethylcyclohexanone (isophoronenitrile, IPN), NH₃ and H₂, wherein the hydrogenation is carried out at from 50 to 200°C and a pressure of from 50 to 300 bar in the presence of a hydrogenation catalyst comprising from 55 to 98% by weight of cobalt, from 0.2 to 15% by weight of phosphorus and from 0.2 to 15% by weight of manganese and also appropriate ruthenium, in each case calculated as oxide and based on the overall weight of the catalyst, wherein the alkali metal content of the hydrogenation catalyst is ≤ 0.03% by weight, calculated as alkali metal oxide and based on the overall weight of the catalyst.

2. The process according to claim 1 carried out as a two-stage process comprising the following steps:
A) reaction of IPN, NH₃ and H₂ at from 20 to 150°C and a pressure of from 50 to 300 bar, if desired in the presence of an imination catalyst;
B) reaction of the reaction mixture obtained in step A) at from 60 to 100°C and a pressure of from 50 to 300 bar in the presence of a hydrogenation catalyst;
where step A) and step B) are carried out either in the same reaction space or in two physically separate reaction spaces.

3. The process according to claim 1 which is carried out in three physically separate reaction spaces and comprises the following reaction steps:
a) reaction of IPN with excess ammonia at from 20 to 150°C and pressures of from 50 to 300 bar in a first reaction space to form essentially 3-cyano-3,5,5-trimethylcyclohexanonimine, if desired over an acidic metal oxide catalyst,
b) hydrogenation of the reaction products obtained in step a) by means of hydrogen at from 60 to 100°C and pressures of from 50 to 300 bar in the presence of excess ammonia over a hydrogenation catalyst in a second reaction space, and
c) hydrogenation of the reaction products obtained in step b) in the presence of hydrogen and ammonia over a hydrogenation catalyst at from 110 to 160°C and pressures of from 50 to 300 bar in a third reaction space.

4. The process according to claim 3, wherein step a) is carried out at from 30 to 130°C and/or pressures of from 100 to 250 bar.

5. The process according to claim 3 or 4, wherein step b) and/or step c) are/is carried out at pressures of from 100 to 250 bar.

6. The process according to any of claims 1 to 5, wherein the alkali metal content of a hydrogenation catalyst is ≤ 0.015% by weight, preferably ≤ 0.01% by weight, calculated as alkali metal oxide and based on the overall weight of the catalyst.

7. A hydrogenation catalyst as defined in claim 1 or 6.

8. The use of a hydrogenation catalyst according to claim 7 for the aminative hydrogenation of isophoronenitrile.

9. A process for producing a hydrogenation catalyst according to claim 7, which comprises:
i) precipitating at least one of the abovementioned transition metals in the form of its carbonate, hydroxide and/or oxide from an aqueous solution comprising at least one water-soluble salt of one of the abovementioned transition metals by means of an aqueous solution comprising at least one substance selected from the group consisting of ammonium carbonate, ammonium hydrogen - carbonate, ammonium carbamate, ammonium oxalate, ammonium malonate, ammonia and urotropin while stirring, if desired with addition of promoters in the form of their water-soluble compounds;
ii) separating off the precipitate obtained in step i);
iiia) drying the resulting precipitate at from 50 to 200°C and milling it to a catalyst powder, or
iiib) slurrying the resulting precipitate and spray drying the resulting suspension at from 100 to 600°C, if desired with addition of promoters in the form of their salts, to give a spray-dried catalyst powder;
iv) calcining the catalyst powder produced in step iiia) or iiib) at from 300 to 1000°C and shaping it to form shaped catalyst bodies, with promoters being able, if desired, to be added in the form of their salts before or during or after shaping to form shaped catalyst bodies;
v) drying and calcining the shaped catalyst bodies produced in step iv);
vi) reducing the dried and calcined shaped catalyst bodies in an H₂/N₂ atmosphere at elevated temperature, with the composition of the H₂/N₂ atmosphere and the temperature being varied;
vii) if desired passivating the reduced shaped catalyst bodies at from 20 to 60°C, with promoters subsequently being applied in the form of their salts to the shaped catalyst bodies if desired.

10. The process according to claim 9, wherein the precipitation in step i) is carried out using an aqueous solution comprising ammonium carbonate

11. The process according to claim 9 or 10, wherein one or more water-soluble Co salts are used in step i)

12. The process according to any of claims 9 to 11, wherein one or more water-soluble Mn salts and one or more water-soluble phosphorus compounds are used as promoters in step i).

## Revendications

1. Procédé pour la préparation de 3-aminométhyl-3,5,5-triméthylcyclohexylamine (isophoronediamine, IPDA) présentant un rapport d'isomères cis/trans d'au moins 70/30 à partir de 3-cyano-3,5,5-triméthylcyclohexanone (isophoronenitrile, IPN), de NH₃ et de H₂, où on hydrogène en présence d'un catalyseur d'hydrogénation contenant 55 à 98% en poids de cobalt, 0,2 à 15% en poids de phosphore et 0,2 à 15% en poids de manganèse ainsi que le cas échéant du ruthénium, à chaque fois calculés sous forme d'oxyde et par rapport au poids total du catalyseur, à des températures de 50 à 200°C et une pression de 50 à 300 bars, **caractérisé en ce que** la teneur en métal alcalin du catalyseur d'hydrogénation est ≤ 0,03% en poids - calculée comme oxyde de métal alcalin et par rapport au poids total du catalyseur.

2. Procédé selon la revendication 1, **caractérisé en ce que** le procédé est réalisé en deux fois, par les étapes suivantes :
A) transformation d'IPN, de NH₃ et de H₂ à des températures de 20 à 150°C et une pression de 50 à 300 bars, le cas échéant en présence d'un catalyseur d'imination ;
B) transformation du mélange réactionnel obtenu dans l'étape A) en présence d'un catalyseur d'hydrogénation à des températures de 60 à 100°C et une pression de 50 à 300 bars ;
où l'étape A) et l'étape B) sont réalisées soit dans le même espace de réaction, soit dans deux espaces de réaction séparés l'un de l'autre dans l'espace.

3. Procédé selon la revendication 1, **caractérisé en ce que** le procédé est réalisé dans trois espaces de réaction séparés les une des autres dans l'espace, présentant les étapes de réaction suivantes :
a) transformation d'IPN dans un premier espace de réaction avec de l'ammoniaque en excès à des températures de 20 à 150°C et des pressions de 50 à 300 bars, essentiellement en 3-cyano-3,5,5-triméthylcyclohexanonimine, le cas échéant sur un catalyseur d'oxyde de métal acide,
b) hydrogénation des produits de réaction obtenus dans l'étape a) dans un deuxième espace de réaction avec de l'hydrogène en présence d'ammoniaque en excès sur un catalyseur d'hydrogénation à des températures de 60 à 100°C et des pressions de 50 à 300 bars, et
c) hydrogénation des produits de réaction obtenus dans l'étape b) dans un troisième espace de réaction en présence d'hydrogène et d'ammoniaque sur un catalyseur d'hydrogénation à des températures de 110 à 160°C et des pressions de 50 à 300 bars

4. Procédé selon la revendication 3, **caractérisé en ce que** l'étape a) est réalisée à des températures de 30 à 130°C et/ou des pressions de 100 à 250 bars.

5. Procédé selon la revendication 3 ou 4,
**caractérisé en ce que** l'étape b) et/ou l'étape c) est réalisée à des pressions de 100 à 250 bars.

6. Procédé selon l'une quelconque des revendications 1 à 5, **caractérisé en ce que** la teneur en métaux alcalins d'un catalyseur d'hydrogénation est ≤ 0,015% en poids, de préférence ≤ 0,01% en poids - calculée comme oxyde de métal alcalin et par rapport au poids total du catalyseur.

7. Catalyseur d'hydrogénation, tel qu'il est défini dans l'une quelconque des revendications 1 ou 6.

8. Utilisation d'un catalyseur d'hydrogénation selon la revendication 7 pour l'hydrogénation avec amination d'isophoronenitrile.

9. Procédé pour la préparation d'un catalyseur d'hydrogénation selon la revendication 7, comprenant les étapes suivantes :
i) précipitation d'au moins un des métaux de transition susmentionnés sous forme de son carbonate, de son hydroxyde et/ou de son oxyde à partir d'une solution aqueuse contenant au moins un sel soluble dans l'eau d'un des métaux de transition mentionnés avec une solution aqueuse contenant au moins une substance choisie dans le groupe formé par le carbonate d'ammonium, l'hydrogénocarbonate d'ammonium, le carbamate d'ammonium, l'oxalate d'ammonium, le malonate d'ammonium, l'ammoniaque et l'urotropine sous agitation - le cas échéant avec addition de promoteurs sous forme de leurs composés solubles dans l'eau ;
ii) séparation du précipité obtenu dans l'étape i) ;
iiia) séchage du précipité ainsi obtenu à des températures de 50 à 200°C et broyage en poudre de catalyseur, ou
iiib) mise en suspension du précipité ainsi obtenu et pulvérisation de la suspension ainsi obtenue à des températures de 100 à 600°C en une poudre pulvérisée de catalyseur - le cas échéant avec addition de promoteurs sous forme de leurs sels ;
iv) calcination de la poudre de catalyseur produite dans l'étape iiia) ou iiib) à des températures de 300 à 1000°C et façonnage en corps façonnés de catalyseur, où on ajoute le cas échéant des promoteurs sous forme de leurs sels avant ou pendant ou après le façonnage en corps façonnés de catalyseur ;
v) séchage et calcination des corps façonnés de catalyseur produits dans l'étape iv),
vi) réduction des corps façonnés de catalyseur séchés et calcinés dans une atmosphère de H₂/N₂ à température élevée, où la composition de l'atmosphère de H₂/N₂ et la température sont variées ;
vii) le cas échéant passivation des corps façonnés réduits à des températures de 20 à 60°C, où on applique le cas échéant ensuite des promoteurs sous forme de leurs sels sur les corps façonnés de catalyseur.

10. Procédé selon la revendication 9, **caractérisé en ce que**, dans l'étape i), on précipite avec une solution aqueuse contenant du carbonate d'ammonium.

11. Procédé selon la revendication 9 ou 10,
**caractérisé en ce qu'**on utilise, dans l'étape i), un ou plusieurs sels de Co solubles dans l'eau.

12. Procédé selon l'une quelconque des revendications 9 à 11, **caractérisé en ce qu'**on utilise, dans l'étape i), comme promoteurs, un ou plusieurs sels de Mn solubles dans l'eau ainsi qu'un ou plusieurs composés phosphorés solubles dans l'eau.
